# EUROPEAN PATENT APPLICATION

(11) **EP 3 378 491 A1**
(43) Date of publication of application: **26.09.2018**
(21) Application number: 16866430.8
(22) Date of filing: 18.11.2016
(51) Int. Cl.: A61K 45/00, A61K 31/381, A61P 1/16, A61P 43/00, C07D 333/36

(54) **PHARMACEUTICAL COMPOSITION FOR TREATMENT OR PROPHYLAXIS OF NASH**

(30) Priority: 20.11.2015 JP 2015227468; 03.06.2016 JP 2016111944
(71) Applicant: UBE Industries, Ltd., Ube-shi, Yamaguchi 755-8633 (JP)
(72) Inventor: NISHIKAWA, Kenji, Ube-shi Yamaguchi 755-8633 (JP); MATSUNAGA, Hirofumi, Ube-shi Yamaguchi 755-8633 (JP); SHINOHARA, Yuuko, Ube-shi Yamaguchi 755-8633 (JP); OKANARI, Eiji, Ube-shi Yamaguchi 755-8633 (JP); USHIYAMA, Shigeru, Ube-shi Yamaguchi 755-8633 (JP); NONOUCHI, Shinpei, Ube-shi Yamaguchi 755-8633 (JP)
(74) Representative: Hoffmann Eitle
(86) International application number: PCT/JP2016/084227
(87) International publication number: WO 2017/086430

(57) **Abstract**

The present invention provides the pharmaceutical use of LPA1 antagonists, for example, α-halogenated thiophene compounds having a specific structure or pharmacologically acceptable salts thereof, as drugs for the treatment and/or prevention of NASH.

A pharmaceutical composition for the treatment and/or prevention of NASH includes, as an active ingredient, an LPA1 antagonist, for example, a compound represented by the general formula (I): 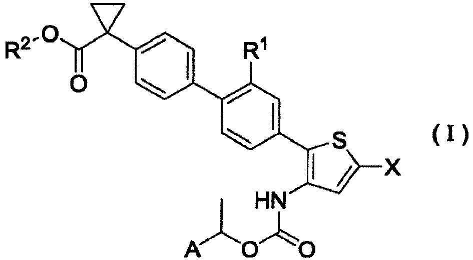 wherein R¹ is a hydrogen atom or a methoxy group, R² is a hydrogen atom or a C₁-C₆ alkyl group, X is a halogen atom, and A is a group selected from the group consisting of groups: 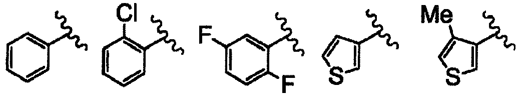 , or a pharmacologically acceptable salt thereof.

## Description

### TECHNICAL FIELD

The present invention relates to a pharmaceutical composition which contains, as an active ingredient, a lysophosphatidic acid receptor (LPA1) antagonist, for example, an α-halogenated thiophene compound having a specific structure or a pharmacologically acceptable salt thereof. The LPA1 antagonist of the present invention, for example, an α-halogenated thiophene compound having a specific structure possesses LPA1 antagonism and is therefore useful for the treatment and/or prevention of LPA-related non-alcoholic steatohepatitis (NASH).

### BACKGROUND ART

Fatty liver is a disease in which neutral fat accumulates in liver. Fatty liver caused by other than excessive alcohol use is called non-alcoholic fatty liver disease (NAFLD). NAFLD is classified into non-alcoholic fatty liver (NAFL) in which there is no pathological hepatocyte disorder, and non-alcoholic steatohepatitis (NASH) which shows inflammation accompanying a hepatocyte disorder. NASH has a clinical condition similar to alcoholic liver disease, and progresses to cirrhosis of the liver and then to hepatocellular carcinoma (Non Patent Literature 1).

NASH is treated with drugs such as antioxidants and insulin sensitizer, but no drugs that show sufficient therapeutic effects have been developed (Non Patent Literature 2).

Lysophosphatidic acid (LPA) is a lipid mediator having various physiological actions. LPA binds to G-protein-coupled receptors (LPA1, LPA2, LPA3, LPA4, LPA5, and LPA6) and transduces signals into cells to control the proliferation, differentiation, survival, migration, adhesion, invasion and morphogenesis of the cells. LPA is known to be involved in various diseases (Non Patent Literature 3).

LPA is involved in various diseases which occur in the liver. It has been reported that LPA promotes the proliferation of stellate cells and the migration of myofibroblasts, these cells play an important role in the process of hepatic fibrosis (Non Patent Literatures 4 and 5). It has been reported that chronic HCV patients have an increased LPA concentration in plasma, and the LPA concentration is correlated with the degree of hepatic fibrosis (Non Patent Literature 6). Further, it has been reported that autotaxin (ATX), which is an LPA-generating enzyme, is increased in the serum of NAFLD patients, and correlated with hepatic steatosis, indicating that the generation of LPA by ATX makes a contribution to steatosis (Non Patent Literature 7).

Some LPA1 antagonistic compounds, ([1,1'-biphenyl]-4-yl)acetic acid derivatives are disclosed in Patent Literatures 1 to 29 and Non Patent Literatures 8 to 11. Patent Literatures 1 to 3, 10 to 13, 15 and 19, and Non Patent Literatures 8 to 10 describe pharmacological data on animal models with diseases such as retinopathy, pulmonary fibrosis, scleroderma and bone marrow damage. However, there are no reports which describe or suggest that LPA1 antagonistic compounds are effective for the treatment or prevention of NASH based on pharmacological data obtained using animal models.

### PRIOR ART DOCUMENTS

### Patent Literature

Patent Literature 1: WO 2010/077882
Patent Literature 2: WO 2010/077883
Patent Literature 3: WO 2010/141761
Patent Literature 4: WO 2010/141768
Patent Literature 5: WO 2011/017350
Patent Literature 6: WO 2011/041461
Patent Literature 7: WO 2011/041462
Patent Literature 8: WO 2011/041694
Patent Literature 9: WO 2011/041729
Patent Literature 10: WO 2011/091167
Patent Literature 11: WO 2011/159632
Patent Literature 12: WO 2011/159633
Patent Literature 13: WO 2011/159635
Patent Literature 14: WO 2012/078593
Patent Literature 15: WO 2012/078805
Patent Literature 16: WO 2012/138648
Patent Literature 17: WO 2012/138797
Patent Literature 18: WO 2013/025733
Patent Literature 19: WO 2013/070879
Patent Literature 20: WO 2013/085824
Patent Literature 21: WO 2013/189862
Patent Literature 22: WO 2013/189864
Patent Literature 23: WO 2013/189865
Patent Literature 24: WO 2014/104372
Patent Literature 25: WO 2014/113485
Patent Literature 26: WO 2014/145873
Patent Literature 27: US 20140200215
Patent Literature 28: WO 2015/066456
Patent Literature 29: CN 104418820

### Non Patent Literature

Non Patent Literature 1: Hepatology, 44 (2006) 865-873
Non Patent Literature 2: Journal of Hepatology, 62 (2015) S65-S75
Non Patent Literature 3: Experimental Cell Research 333, (2015) 171-177
Non Patent Literature 4: Biochemical and Biophysical Research Communications, 248 (1998) 436-440
Non Patent Literature 5: Journal of Biomedical Science, 10 (2003) 352-358
Non Patent Literature 6: Journal of Clinical Gastroenterology, 41 (2007) 616-623
Non Patent Literature 7: Obesity, 23 (2015) 965-972
Non Patent Literature 8: The Journal of Pharmacology and Experimental Therapeutics, 336 (2011) 693-700
Non Patent Literature 9: British Journal of Pharmacology, 160 (2010) 1699-1713
Non Patent Literature 10: Arthritis & Rheumatism, 63 (2011) 1405-1415
Non Patent Literature 11: Journal of Medicinal Chemistry, 55 (2012) 7920-7939

### SUMMARY OF INVENTION

### Problems to be solved by the Invention

The present inventors carried out researches directed to developing potent NASH treatment or preventive drugs. As a result, the present inventors have found that α-halogenated thiophene compounds with a specific structure have superior LPA1 antagonism and useful as drugs for the treatment and/or prevention of NASH, thereby completing the present invention.

The present invention provides the pharmaceutical use of LPA1 antagonists, for example, α-halogenated thiophene compounds having a specific structure or pharmacologically acceptable salts thereof, as drugs for the treatment and/or prevention (preferably, for the treatment) of NASH.

### Means for solving the Problems

The present invention provides the following.
(1) A pharmaceutical composition for the treatment and/or prevention of NASH, comprising an LPA1 antagonist as an active ingredient.
(2) The pharmaceutical composition for the treatment and/or prevention of NASH according to (1), wherein the LPA1 antagonist is an α-halogenated thiophene compound represented by the general formula (I): 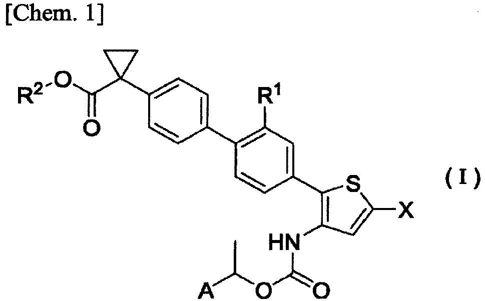 wherein
   R¹ is a hydrogen atom or a methoxy group,
   R² is a hydrogen atom or a C₁-C₆ alkyl group,
   X is a halogen atom, and
   A is selected from the group consisting of: 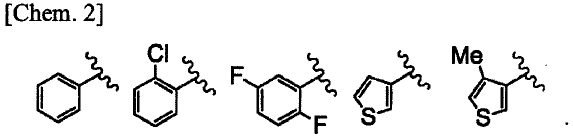
   or a pharmacologically acceptable salt thereof.
(3) The pharmaceutical composition for the treatment and/or prevention of NASH, comprising an α-halogenated thiophene compound described in (2) wherein X in the general formula (I) is a fluorine atom or a chlorine atom, or a pharmacologically acceptable salt thereof as an active ingredient.
(4) The pharmaceutical composition for the treatment and/or prevention of NASH, comprising an α-halogenated thiophene compound described in (3) wherein R¹ in the general formula (I) is a hydrogen atom, or a pharmacologically acceptable salt thereof as an active ingredient.
(5) The pharmaceutical composition for the treatment and/or prevention of NASH, comprising an α-halogenated thiophene compound described in (3) wherein R¹ in the general formula (I) is a methoxy group, or a pharmacologically acceptable salt thereof as an active ingredient.
(6) A pharmaceutical composition for the treatment and/or prevention of NASH, comprising (R)-1-[4'-(5-chloro-3-{[(1-phenylethoxy)carbonyl]amino}thiophen-2-yl)-2'-methoxy-[1 ,1'-biphenyl]-4-yl]cyclopropanecarboxylic acid, or a pharmacologically acceptable salt thereof as an active ingredient.
(7) A pharmaceutical composition for the treatment and/or prevention of NASH, comprising (R)-1-{4'-[5-chloro-3-({[1-(2,5-difluorophenyl)ethoxy]carbonyl}amino)thiophen-2-yl]-2'-methoxy-[1,1'-biphenyl]-4-yl}cyclopropanecarboxylic acid, or a pharmacologically acceptable salt thereof as an active ingredient.
(8) A pharmaceutical composition for the treatment and/or prevention of NASH, comprising (R)-1-{4'-[3-({[1-(2-chlorophenyl)ethoxy]carbonyl}amino)-5-fluorothiophen-2-yl]-2'-methoxy-[1,1'-biphenyl]-4-yl}cyclopropanecarboxylic acid, or a pharmacologically acceptable salt thereof as an active ingredient.
(9) A pharmaceutical composition for the treatment and/or prevention of NASH, comprising (R)-1-{4'-[5-chloro-3-(([1-(thiophen-3-yl)ethoxy]carbonyl}amino)thiophen-2-yl]-[1,1'-biphenyl]-4-yl}cyclopropanecarboxylic acid, or a pharmacologically acceptable salt thereof as an active ingredient.
(10) A pharmaceutical composition for the treatment and/or prevention of NASH, comprising
   (R)-1-{4'-[5-fluoro-3-({[1-(4-methylthiophen-3-yl)ethoxy]carbonyl}amino)thiophen-2-yl]-[1,1'-biphenyl]-4-yl}cyclopropanecarboxylic acid, or a pharmacologically acceptable salt thereof as an active ingredient.
(11) A method for the treatment and/or prevention of NASH, comprising administering to a subject in need thereof an effective dose of an LPA1 antagonist, for example, the α-halogenated thiophene compound of the general formula (I) or a pharmacologically acceptable salt thereof described in any one of (2) to (10).
(12) An LPA1 antagonist, for example, the α-halogenated thiophene compound of the general formula (I) or a pharmacologically acceptable salt thereof described in any one of (2) to (10), for use in the treatment and/or prevention of NASH.
(13) Use of an LPA1 antagonist, for example, the α-halogenated thiophene compound of the general formula (I) or a pharmacologically acceptable salt thereof described in any one of (2) to (10), for the treatment and/or prevention of NASH.
(14) Use of an LPA1 antagonist, for example, the α-halogenated thiophene compound of the general formula (I) described in any one of (2) to (10), or a pharmacologically acceptable salt thereof, for the production of a pharmaceutical for the treatment and/or prevention of NASH.

The LPA1 antagonists of the present invention are not particularly limited as long as having antagonism at LPA1, and examples thereof include those compounds represented by the general formula (I) of the present invention. For the reasons that the LPA1 antagonists attain potent LPA1 antagonism and exhibit high effects in the treatment and/or prevention of NASH, the IC₅₀ for LPA1 antagonism is preferably 0.001 nM to 500 nM, and more preferably 0.001 nM to 100 nM.

The present invention also pertains to a method for screening compounds for the treatment and/or prevention of NASH, which includes evaluating the antagonism of a candidate compound at LPA1. For example, the method may be such that the IC₅₀ for the LPA1 antagonism of candidate compounds is measured and those compounds having a value of IC₅₀ of 0.001 nM to 1000 nM, preferably 0.001 nM to 500 nM, and more preferably 0.001 nM to 100 nM are screened out for use as compounds for the treatment and/or prevention of NASH.

The above IC₅₀ for the LPA1 antagonism is determined by the method described in Test Example 1 of the present application.

Specific examples of the compounds represented by the general formula (I) of the present invention include compounds described in Table 1 below. In Table 1, group A is a group represented by any of the formulae A1 to A5 below, Me represents a methyl group, Et represents an ethyl group, n-Pr represents an n-propyl group, iso-Pr represents an isopropyl group, F represents a fluorine atom, Cl represents a chlorine atom, Br represents a bromine atom, OMe represents a methoxy group, and "racemic" and "(R)-" represent the configuration of the carbon atom marked with "*" in the general formula (I) below.

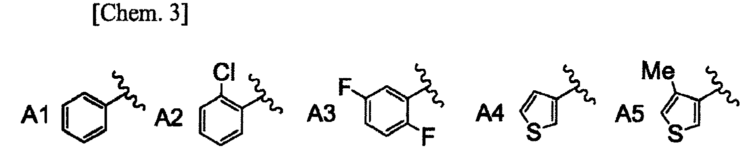

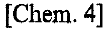

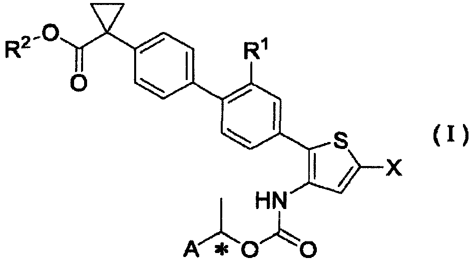

**[Table 1]**

| Compound No. | R¹ | X | A | R² | Configuration |
|---|---|---|---|---|---|
| I-1 | H | Br | A1 | Et | racemic |
| I-2 | H | Br | A1 | Et | (R)- |
| I-3 | H | Br | A1 | H | racemic |
| I-4 | H | Br | A1 | H | (R)- |
| I-5 | H | Br | A2 | Et | racemic |
| I-6 | H | Br | A2 | Et | (R)- |
| I-7 | H | Br | A2 | H | racemic |
| I-8 | H | Br | A2 | H | (R)- |
| I-9 | H | Br | A3 | Et | racemic |
| I-10 | H | Br | A3 | Et | (R)- |
| I-11 | H | Br | A3 | H | racemic |
| I-12 | H | Br | A3 | H | (R)- |
| I-13 | H | Br | A4 | Et | racemic |
| I-14 | H | Br | A4 | Et | (R)- |
| I-15 | H | Br | A4 | H | racemic |
| I-16 | H | Br | A4 | H | (R)- |
| I-17 | H | Br | A5 | Et | racem ic |
| I-18 | H | Br | A5 | Et | (R)- |
| I-19 | H | Br | A5 | H | racemic |
| I-20 | H | Br | A5 | H | (R)- |
| I-21 | H | Cl | A1 | iso-Pr | racemic |
| I-22 | H | Cl | A1 | iso-Pr | (R)- |
| I-23 | H | Cl | A1 | n-Pr | racemic |
| I-24 | H | Cl | A1 | n-Pr | (R)- |
| I-25 | H | Cl | A1 | Et | racemic |
| I-26 | H | Cl | A1 | Et | (R)- |
| I-27 | H | Cl | A1 | Me | racemic |
| I-28 | H | Cl | A1 | Me | (R)- |
| I-29 | H | Cl | A1 | H | racemic |
| I-30 | H | Cl | A1 | H | (R)- |
| I-31 | H | Cl | A2 | iso-Pr | racemic |
| I-32 | H | Cl | A2 | iso-Pr | (R)- |
| I-33 | H | Cl | A2 | n-Pr | racemic |
| I-34 | H | Cl | A2 | n-Pr | (R)- |
| I-35 | H | Cl | A2 | Et | racemic |
| I-36 | H | Cl | A2 | Et | (R)- |
| I-37 | H | Cl | A2 | Me | racemic |
| I-38 | H | Cl | A2 | Me | (R)- |
| I-39 | H | Cl | A2 | H | racemic |
| I-40 | H | Cl | A2 | H | (R)- |
| I-41 | H | Cl | A3 | iso-Pr | racemic |
| I-42 | H | Cl | A3 | iso-Pr | (R)- |
| I-43 | H | Cl | A3 | n-Pr | racemic |
| I-44 | H | Cl | A3 | n-Pr | (R)- |
| I-45 | H | Cl | A3 | Et | racemic |
| I-46 | H | Cl | A3 | Et | (R)- |
| I-47 | H | Cl | A3 | Me | racemic |
| I-48 | H | Cl | A3 | Me | (R)- |
| I-49 | H | Cl | A3 | H | racemic |
| I-50 | H | Cl | A3 | H | (R)- |
| I-51 | H | Cl | A4 | iso-Pr | racemic |
| I-52 | H | Cl | A4 | iso-Pr | (R)- |
| I-53 | H | Cl | A4 | n-Pr | racemic |
| I-54 | H | Cl | A4 | n-Pr | (R)- |
| I-55 | H | Cl | A4 | Et | racemic |
| I-56 | H | Cl | A4 | Et | (R)- |
| I-57 | H | Cl | A4 | Me | racemic |
| I-58 | H | Cl | A4 | Me | (R)- |
| I-59 | H | Cl | A4 | H | racemic |
| I-60 | H | Cl | A4 | H | (R)- |
| I-61 | H | Cl | A5 | iso-Pr | racemic |
| I-62 | H | Cl | A5 | iso-Pr | (R)- |
| I-63 | H | Cl | A5 | n-Pr | racemic |
| I-64 | H | Cl | A5 | n-Pr | (R)- |
| I-65 | H | Cl | A5 | Et | racemic |
| I-66 | H | Cl | A5 | Et | (R)- |
| I-67 | H | Cl | A5 | Me | racemic |
| I-68 | H | Cl | A5 | Me | (R)- |
| I-69 | H | Cl | A5 | H | racemic |
| I-70 | H | Cl | A5 | H | (R)- |
| I-71 | H | F | A1 | iso-Pr | racemic |
| I-72 | H | F | A1 | iso-Pr | (R)- |
| I-73 | H | F | A1 | n-Pr | racemic |
| I-74 | H | F | A1 | n-Pr | (R)- |
| I-75 | H | F | A1 | Et | racemic |
| I-76 | H | F | A1 | Et | (R)- |
| I-77 | H | F | A1 | Me | racemic |
| I-78 | H | F | A1 | Me | (R)- |
| I-79 | H | F | A1 | H | racemic |
| I-80 | H | F | A1 | H | (R)- |
| I-81 | H | F | A2 | iso-Pr | racemic |
| I-82 | H | F | A2 | iso-Pr | (R)- |
| I-83 | H | F | A2 | n-Pr | racemic |
| I-84 | H | F | A2 | n-Pr | (R)- |
| I-85 | H | F | A2 | Et | racemic |
| I-86 | H | F | A2 | Et | (R)- |
| I-87 | H | F | A2 | Me | racemic |
| I-88 | H | F | A2 | Me | (R)- |
| I-89 | H | F | A2 | H | racemic |
| I-90 | H | F | A2 | H | (R)- |
| I-91 | H | F | A3 | iso-Pr | racemic |
| I-92 | H | F | A3 | iso-Pr | (R)- |
| I-93 | H | F | A3 | n-Pr | racemic |
| I-94 | H | F | A3 | n-Pr | (R)- |
| I-95 | H | F | A3 | Et | racemic |
| I-96 | H | F | A3 | Et | (R)- |
| I-97 | H | F | A3 | Me | racemic |
| I-98 | H | F | A3 | Me | (R)- |
| I-99 | H | F | A3 | H | racemic |
| I-100 | H | F | A3 | H | (R)- |
| I-101 | H | F | A4 | iso-Pr | racemic |
| I-102 | H | F | A4 | iso-Pr | (R)- |
| I-103 | H | F | A4 | n-Pr | racemic |
| I-104 | H | F | A4 | n-Pr | (R)- |
| I-105 | H | F | A4 | Et | racemic |
| I-106 | H | F | A4 | Et | (R)- |
| I-107 | H | F | A4 | Me | racemic |
| I-108 | H | F | A4 | Me | (R)- |
| I-109 | H | F | A4 | H | racemic |
| I-110 | H | F | A4 | H | (R)- |
| I-111 | H | F | A5 | iso-Pr | racemic |
| I-112 | H | F | A5 | iso-Pr | (R)- |
| I-113 | H | F | A5 | n-Pr | racemic |
| I-114 | H | F | A5 | n-Pr | (R)- |
| I-115 | H | F | A5 | Et | racemic |
| I-116 | H | F | A5 | Et | (R)- |
| I-117 | H | F | A5 | Me | racemic |
| I-118 | H | F | A5 | Me | (R)- |
| I-119 | H | F | A5 | H | racemic |
| I-120 | H | F | A5 | H | (R)- |
| I-121 | OMe | Br | A1 | Et | racemic |
| I-122 | OMe | Br | A1 | Et | (R)- |
| I-123 | OMe | Br | A1 | H | racemic |
| I-124 | OMe | Br | A1 | H | (R)- |
| I-125 | OMe | Br | A2 | Et | racemic |
| I-126 | OMe | Br | A2 | Et | (R)- |
| I-127 | OMe | Br | A2 | H | racemic |
| I-128 | OMe | Br | A2 | H | (R)- |
| I-129 | OMe | Br | A3 | Et | racemic |
| I-130 | OMe | Br | A3 | Et | (R)- |
| I-131 | OMe | Br | A3 | H | racemic |
| I-132 | OMe | Br | A3 | H | (R)- |
| I-133 | OMe | Br | A4 | Et | racemic |
| I-134 | OMe | Br | A4 | Et | (R)- |
| I-135 | OMe | Br | A4 | H | racemic |
| I-136 | OMe | Br | A4 | H | (R)- |
| I-137 | OMe | Br | A5 | Et | racemic |
| I-138 | OMe | Br | A5 | Et | (R)- |
| I-139 | OMe | Br | A5 | H | racemic |
| I-140 | OMe | Br | A5 | H | (R)- |
| I-141 | OMe | Cl | A1 | iso-Pr | racemic |
| I-142 | OMe | Cl | A1 | iso-Pr | (R)- |
| I-143 | OMe | Cl | A1 | n-Pr | racemic |
| I-144 | OMe | Cl | A1 | n-Pr | (R)- |
| I-145 | OMe | Cl | A1 | Et | racemic |
| I-146 | OMe | Cl | A1 | Et | (R)- |
| I-147 | OMe | Cl | A1 | Me | racemic |
| I-148 | OMe | Cl | A1 | Me | (R)- |
| I-149 | OMe | Cl | A1 | H | racemic |
| I-150 | OMe | Cl | A1 | H | (R)- |
| I-151 | OMe | Cl | A2 | iso-Pr | racemic |
| I-152 | OMe | Cl | A2 | iso-Pr | (R)- |
| I-153 | OMe | Cl | A2 | n-Pr | racemic |
| I-154 | OMe | Cl | A2 | n-Pr | (R)- |
| I-155 | OMe | Cl | A2 | Et | racemic |
| I-156 | OMe | Cl | A2 | Et | (R)- |
| I-157 | OMe | Cl | A2 | Me | racemic |
| I-158 | OMe | Cl | A2 | Me | (R)- |
| I-159 | OMe | Cl | A2 | H | racemic |
| I-160 | OMe | Cl | A2 | H | (R)- |
| I-161 | OMe | Cl | A3 | iso-Pr | racemic |
| I-162 | OMe | Cl | A3 | iso-Pr | (R)- |
| I-163 | OMe | Cl | A3 | n-Pr | racemic |
| I-164 | OMe | Cl | A3 | n-Pr | (R)- |
| I-165 | OMe | Cl | A3 | Et | racemic |
| I-166 | OMe | Cl | A3 | Et | (R)- |
| I-167 | OMe | Cl | A3 | Me | racemic |
| I-168 | OMe | Cl | A3 | Me | (R)- |
| I-169 | OMe | Cl | A3 | H | racemic |
| I-170 | OMe | Cl | A3 | H | (R)- |
| I-171 | OMe | Cl | A4 | iso-Pr | racemic |
| I-172 | OMe | Cl | A4 | iso-Pr | (R)- |
| I-173 | OMe | Cl | A4 | n-Pr | racemic |
| I-174 | OMe | Cl | A4 | n-Pr | (R)- |
| I-175 | OMe | Cl | A4 | Et | racemic |
| I-176 | OMe | Cl | A4 | Et | (R)- |
| I-177 | OMe | Cl | A4 | Me | racemic |
| I-178 | OMe | Cl | A4 | Me | (R)- |
| I-179 | OMe | Cl | A4 | H | racemic |
| I-180 | OMe | Cl | A4 | H | (R)- |
| I-181 | OMe | Cl | A5 | iso-Pr | racemic |
| I-182 | OMe | Cl | A5 | iso-Pr | (R)- |
| I-183 | OMe | Cl | A5 | n-Pr | racemic |
| I-184 | OMe | Cl | A5 | n-Pr | (R)- |
| I-185 | OMe | Cl | A5 | Et | racemic |
| I-186 | OMe | Cl | A5 | Et | (R)- |
| I-187 | OMe | Cl | A5 | Me | racemic |
| I-188 | OMe | Cl | A5 | Me | (R)- |
| I-189 | OMe | Cl | A5 | H | racemic |
| I-190 | OMe | Cl | A5 | H | (R)- |
| I-191 | OMe | F | A1 | iso-Pr | racemic |
| I-192 | OMe | F | A1 | iso-Pr | (R)- |
| I-193 | OMe | F | A1 | n-Pr | racemic |
| I-194 | OMe | F | A1 | n-Pr | (R)- |
| I-195 | OMe | F | A1 | Et | racemic |
| I-196 | OMe | F | A1 | Et | (R)- |
| I-197 | OMe | F | A1 | Me | racemic |
| I-198 | OMe | F | A1 | Me | (R)- |
| I-199 | OMe | F | A1 | H | racemic |
| I-200 | OMe | F | A1 | H | (R)- |
| I-201 | OMe | F | A2 | iso-Pr | racemic |
| I-202 | OMe | F | A2 | iso-Pr | (R)- |
| I-203 | OMe | F | A2 | n-Pr | racemic |
| I-204 | OMe | F | A2 | n-Pr | (R)- |
| I-205 | OMe | F | A2 | Et | racemic |
| I-206 | OMe | F | A2 | Et | (R)- |
| I-207 | OMe | F | A2 | Me | racemic |
| I-208 | OMe | F | A2 | Me | (R)- |
| I-209 | OMe | F | A2 | H | racemic |
| I-210 | OMe | F | A2 | H | (R) - |
| I-211 | OMe | F | A3 | iso-Pr | racemic |
| I-212 | OMe | F | A3 | iso-Pr | (R)- |
| I-213 | OMe | F | A3 | n-Pr | racemic |
| I-214 | OMe | F | A3 | n-Pr | (R)- |
| I-215 | OMe | F | A3 | Et | racemic |
| I-216 | OMe | F | A3 | Et | (R)- |
| I-217 | OMe | F | A3 | Me | racemic |
| I-218 | OMe | F | A3 | Me | (R)- |
| I-219 | OMe | F | A3 | H | racemic |
| I-220 | OMe | F | A3 | H | (R)- |
| I-221 | OMe | F | A4 | iso-Pr | racemic |
| I-222 | OMe | F | A4 | iso-Pr | (R)- |
| I-223 | OMe | F | A4 | n-Pr | racemic |
| I-224 | OMe | F | A4 | n-Pr | (R)- |
| I-225 | OMe | F | A4 | Et | racemic |
| I-226 | OMe | F | A4 | Et | (R)- |
| I-227 | OMe | F | A4 | Me | racemic |
| I-228 | OMe | F | A4 | Me | (R)- |
| I-229 | OMe | F | A4 | H | racemic |
| I-230 | OMe | F | A4 | H | (R)- |
| I-231 | OMe | F | A5 | iso-Pr | racemic |
| I-232 | OMe | F | A5 | iso-Pr | (R)- |
| I-233 | OMe | F | A5 | n-Pr | racemic |
| I-234 | OMe | F | A5 | n-Pr | (R)- |
| I-235 | OMe | F | A5 | Et | racemic |
| I-236 | OMe | F | A5 | Et | (R)- |
| I-237 | OMe | F | A5 | Me | racemic |
| I-238 | OMe | F | A5 | Me | (R)- |
| I-239 | OMe | F | A5 | H | racemic |
| I-240 | OMe | F | A5 | H | (R)- |

### Effects of Invention

The LPA1 antagonists of the present invention, for example, α-halogenated thiophene compounds represented by the general formula (I) or pharmacologically acceptable salts thereof have potent LPA1 antagonism and are therefore useful as drugs for the treatment and/or prevention of NASH.

### MODE FOR CARRYING OUT THE INVENTION

Preferred embodiments of each of the substituents in the compounds represented by the general formula (I) will be described below.

Examples of the "halogen atoms" represented by X include fluorine atom, chlorine atom, bromine atom and iodine atom.

Preferably, the "halogen atom" represented by X is a fluorine atom, a chlorine atom or a bromine atom, and is more preferably a fluorine atom or a chlorine atom.

Examples of the "C₁-C₆ alkyl groups" represented by R² include linear or branched C₁-C₆ alkyl groups such as methyl group, ethyl group, propyl group, isopropyl group, butyl group, isobutyl group, sec-butyl group, tert-butyl group, pentyl group, isopentyl group, neopentyl group, tert-pentyl group, 1-methylbutyl group, 2-methylbutyl group, 1-ethylpropyl group, 1,2-dimethylpropyl group, hexyl group, 1-methylpentyl group, 2-methylpentyl group, 3-methylpentyl group, 4-methylpentyl group, 1-ethylbutyl group, 2-ethylbutyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1-ethyl-1-methylpropyl group, 1-ethyl-2-methylpropyl group, 1,1,2-trimethylpropyl group and 1,2,2-trimethylpropyl group.

The "C₁-C₆ alkyl group" represented by R² is preferably a C₁-C₃ alkyl group, and more preferably an ethyl group.

R² is preferably a hydrogen atom or an ethyl group, and more preferably a hydrogen atom.

In the case where the compounds represented by the general formula (I) of the present invention have optical isomers, geometric isomers and rotational isomers, these isomers are within the scope of the present invention. Further, in the case where proton tautomerism is present, these tautomers are also within the scope of the present invention.

In the general formula (I), the group represented by: 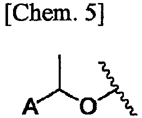 is preferably the following group:

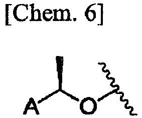

The compound represented by the general formula (I) of the present invention in which R² is a hydrogen atom may be treated with a base to form a pharmacologically acceptable basic salt. Examples of such salts include metal salts such as sodium salts, potassium salts, calcium salts and magnesium salts; inorganic salts such as ammonium salts; and organic amine salts such as triethylamine salts and guanidine salts.

The compounds represented by the general formula (I) of the present invention, or pharmacologically acceptable salts thereof, may form hydrates or solvates, of which each and mixtures are also within the scope of the present invention.

One or more kinds of the atoms constituting the salts represented by the formula (I) of the present invention may have atomic isotopes in an unnatural proportion. Examples of the atomic isotopes include deuterium (²H), tritium (³H), carbon-14 (¹⁴C), fluorine-18 (¹⁸F), sulfur-35 (³⁵S) and iodine-125 (¹²¹I). Such compounds are useful as treatment or preventive medicaments, research reagents such as assay reagents, and diagnostic agents such as in vivo diagnostic imaging agents. All the isotopic variants of the salts represented by the formula (I) of the present invention are within the scope of the present invention irrespective of whether or not they are radioactive.

The compounds represented by the general formula (I) of the present invention, or pharmacologically acceptable salts thereof are preferably compounds Nos. I-56, I-60, I-116, I-120, I-146, I-150, I-170, 1-206 and 1-210, or pharmacologically acceptable salts thereof; are particularly preferably compounds Nos. I-60, I-120, I-150, I-170 and I-210, or pharmacologically acceptable salts thereof; and are especially preferably compounds Nos. I-150 and I-170, or pharmacologically acceptable salts thereof, for the reasons that they attain potent LPA1 antagonism and exhibit high effects in the treatment and/or prevention of NASH.

The compounds represented by the general formula (I) of the present invention, or pharmacologically acceptable salts thereof, may be produced by known methods, for example, by or in accordance with the method described in Patent Literature 24 (WO 2014/104372).

A pharmaceutical composition of the present invention includes the LPA1 antagonist of the present invention, for example, a compound represented by the general formula (I) or a pharmacologically acceptable salt thereof, and may further include other pharmaceutically active ingredients, for example, substances used for the treatment and/or prevention of NASH.

While still achieving the effects of the present invention, the pharmaceutical composition of the present invention may contain pharmacologically acceptable additives such as excipients, lubricants, binders, disintegrants, emulsifiers, stabilizers, flavoring agents or diluents, and may be administered orally or parenterally (such as by intravenous administration, intramuscular administration, intraperitoneal administration, percutaneous administration, intratracheal administration, intracutaneous administration or subcutaneous administration) in forms such as tablets, capsules, powders, syrups, granules, fine granules, pills, suspensions, emulsions, percutaneous absorption preparations, suppositories, ointments, lotions, inhalants or injection products.

When the LPA1 antagonists of the present invention, for example, compounds represented by the general formula (I) or pharmacologically acceptable salts thereof, are used as medicaments for the treatment and/or prevention of NASH, the antagonists themselves (as ingredients) may be administered as such or may be administered orally or parenterally (such as by intravenous administration, intramuscular administration, intraperitoneal administration, percutaneous administration, intratracheal administration, intracutaneous administration or subcutaneous administration) in forms such as tablets, capsules, powders, syrups, granules, fine granules, pills, suspensions, emulsions, percutaneous absorption preparations, suppositories, ointments, lotions, inhalants or injection products, which are manufactured by mixing the antagonists with appropriate pharmacologically acceptable additives such as excipients, lubricants, binders, disintegrants, emulsifiers, stabilizers, flavoring agents or diluents, while still achieving the effects of the present invention.

These preparations are manufactured by known methods using pharmacologically acceptable additives such as excipients, lubricants, binders, disintegrants, emulsifiers, stabilizers, flavoring agents or diluents.

Examples of the excipients include organic excipients or inorganic excipients. Examples of the organic excipients include sugar derivatives such as lactose, sucrose, glucose, mannitol or sorbitol; starch derivatives such as corn starch, potato starch, α-starch or dextrin; cellulose derivatives such as crystalline cellulose; gum arabic; dextran; or pullulan. Examples of the inorganic excipients include light anhydrous silicic acid; or sulfate salts such as calcium sulfate.

Examples of the lubricants include stearic acid; metal salts of stearic acid such as calcium stearate or magnesium stearate; talc; colloidal silica; waxes such as bees wax or spermaceti wax; boric acid; adipic acid; sulfate salts such as sodium sulfate; glycol; fumaric acid; sodium benzoate; D, L-leucine; sodium laurylsulfate; silicic acids such as silicic anhydride or silicic acid hydrate; or starch derivatives listed as the excipients above.

Examples of the binders include hydroxypropylcellulose, hydroxypropylmethylcellulose, polyvinylpyrrolidone, macrogol or compounds listed as the excipients above.

Examples of the disintegrants include cellulose derivatives such as low-substituted hydroxypropylcellulose, carboxymethylcellulose, carboxymethylcellulose calcium or internally-crosslinked carboxymethylcellulose calcium; crosslinked polyvinylpyrrolidone; or chemically modified starch or cellulose derivatives such as carboxymethyl starch or sodium carboxymethyl starch.

Examples of the emulsifiers include colloidal clays such as bentonite or bee gum; anionic surfactants such as sodium laurylsulfate; cationic surfactants such as benzalkonium chloride; or nonionic surfactants such as polyoxyethylene alkyl ether, polyoxyethylene sorbitan fatty acid ester or sucrose fatty acid ester.

Examples of the stabilizers include p-hydroxybenzoate esters such as methylparaben or propylparaben; alcohols such as chlorobutanol, benzyl alcohol or phenylethyl alcohol; benzalkonium chlorides; phenols such as phenol or cresol; thimerosal; acetic anhydride; or sorbic acid.

Examples of the flavoring agents include sweeteners such as saccharin sodium or aspartame; acidulants such as citric acid, malic acid or tartaric acid; or flavors such as menthol, lemon extract or orange extract.

The diluents are compounds usually used for dilution. Examples thereof include lactose, mannitol, glucose, sucrose, calcium sulfate, hydroxypropylcellulose, microcrystalline cellulose, water, ethanol, polyethylene glycol, propylene glycol, glycerol, starch, polyvinylpyrrolidone or mixtures thereof.

In the present invention, for the treatment and/or prevention of NASH, the pharmaceutical composition of the present invention, or the LPA1 antagonist, for example, a compound represented by the general formula (I) or a pharmacologically acceptable salt thereof, is administered in an effective dose to a subject in need thereof. Examples of the subjects include animals, for example, mammals such as humans. Typical subjects are NASH patients.

The effective dose of the LPA1 antagonist of the present invention, for example, a compound represented by the general formula (I) or a pharmacologically acceptable salt thereof, may vary depending on conditions such as symptoms, ages and body weights of the subjects. In the case of oral administration, the lower and upper limit doses per administration may be 0.001 mg/kg (preferably 0.01 mg/kg) and 20 mg/kg (preferably 10 mg/kg), respectively. In the case of parenteral administration, the lower and upper limit doses per administration may be 0.0001 mg/kg (preferably 0.0005 mg/kg) and 10 mg/kg (preferably 5 mg/kg), respectively. In both cases, the number of administrations for adults may be 1 to 6 per day depending on symptoms.

### EXAMPLES

The present invention will be described in further detail hereinbelow by presenting production examples (Production Examples 1 to 9), test examples (Test Examples 1 to 5), and preparation examples (Preparation Examples 1 to 3). These examples are only served to help the understanding of the present invention and do not intend to limit the scope of the present invention.

Unless otherwise mentioned, Me and Et in the chemical structures indicate a methyl group and an ethyl group, respectively.

### [Production Examples]

### (Production Example 1)

### (R)-1-{4'-[5-chloro-3-({[1-(thiophen-3-yl)ethoxy]carbonyl}amino)thiophen-2-yl]-[1,1'-biphenyl]-4-yl}cyclopropanecarboxylic acid ethyl ester (Compound No. I-56)

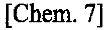

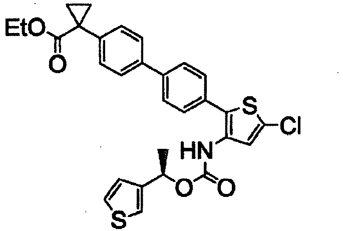

The compound was produced in accordance with the method described in Example 111 of Patent Literature 24 (WO 2014/104372).
Mass spectrum (DUIS⁻, m/z): 550 [M-1]⁻.
¹H-NMR spectrum (400 MHz, CDCl₃) δ: 7.69-7.63 (2H, m), 7.63-7.51 (3H, m), 7.48-7.40 (4H, m), 7.31 (1H, dd, J = 5.0, 2.9 Hz), 7.28-7.26 (1H, m), 7.11 (1H, dd, J = 5.0, 1.3 Hz), 6.72 (1H, s), 5.99 (1H, q, J = 6.6 Hz), 4.13 (2H, q, J = 7.1 Hz), 1.64 (2H, dd, J = 7.0, 4.0 Hz), 1.62 (3H, d, J = 6.5 Hz), 1.23 (2H, dd, J = 7.0, 4.0 Hz), 1.19 (3H, t, J = 7.1 Hz).

### (Production Example 2)

### (R)-1-[4'-(5-chloro-3-{[(1-phenylethoxy)carbonyl]amino}thiophen-2-yl)-2'-methoxy-[1 ,1'-biphenyl]-4-yl]cyclopropanecarboxylic acid ethyl ester (Compound No. 1-146)

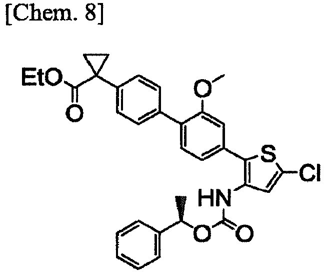

The compound was produced in accordance with the method described in Example 51 of Patent Literature 24 (WO 2014/104372).
Mass spectrum (CI, m/z): 575 [M]⁺.
¹H-NMR spectrum (400 MHz, DMSO-d₆) δ: 9.39 (1H, brs), 7.40-7.26 (10H, m), 7.23-7.16 (2H, m), 7.10 (1H, dd, J = 7.8, 1.6 Hz), 5.75 (1H, q, J = 6.4 Hz), 4.06 (2H, q, J = 7.1 Hz), 3.73 (3H, s), 1.56-1.41 (5H, m), 1.23 (2H, dd, J = 7.0, 4.0 Hz), 1.13 (3H, t, J = 7.0 Hz).

### (Production Example 3)

### (R)-1-{4'-[5-fluoro-3-({[1-(4-methylthiophen-3-yl)ethoxy]carbonyl}amino)thiophen-2-yl]-[1,1'-biphenyl]-4-yl}cyclopropanecarboxylic acid ethyl ester (Compound No. 1-116)

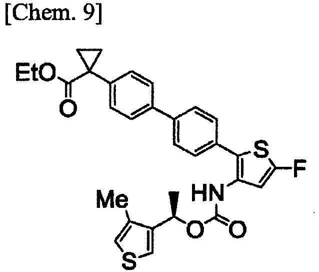

The compound was produced in accordance with the method described in Example 137 of Patent Literature 24 (WO 2014/104372).
Mass spectrum (DUIS⁻, m/z): 548 [M-1]⁻.
¹H-NMR spectrum (400 MHz, DMSO-d₆) δ: 9.31 (1H, brs), 7.74-7.68 (2H, m), 7.66-7.61 (2H, m), 7.57-7.40 (5H, m), 7.17-7.13 (1H, m), 6.83 (1H, brs), 5.74 (1H, q, J = 6.5 Hz), 4.05 (2H, q, J = 7.2 Hz), 2.17 (3H, brs), 1.60-1.43 (3H, m), 1.51 (2H, dd, J = 6.8, 4.0 Hz), 1.23 (2H, dd, J = 7.1, 4.1 Hz), 1.11 (3H, t, J = 7.1 Hz).

### (Production Example 4)

### (R)-1-{4'-[3-({[1-(2-chlorophenyl)ethoxy]carbonyl}amino)-5-fluorothiophen-2-yl]-2'-methoxy-[1,1'-biphenyl]-4-yl}cyclopropanecarboxylic acid ethyl ester (Compound No. I-206)

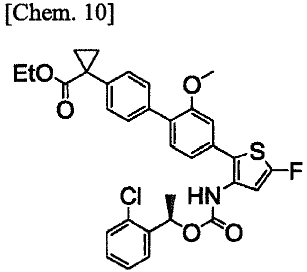

The compound was produced in accordance with the method described in Example 92 of Patent Literature 24 (WO 2014/104372).
Mass spectrum (EI, m/z): 593 [M]⁺.
¹H-NMR spectrum (400 MHz, CDCl₃) δ: 7.52-7.48 (2H, m), 7.43-7.17 (9H, m), 7.06 (1H, dd, J = 7.8, 1.6 Hz), 6.97 (1H, d, J = 1.6 Hz), 6.23 (1H, q, J = 6.7 Hz), 4.13 (2H, q, J = 7.1 Hz), 3.82 (3H, s), 1.63 (2H, dd, J = 6.9, 3.9 Hz), 1.57 (3H, d, J = 6.7 Hz), 1.24 (2H, dd, J = 7.0, 4.0 Hz), 1.20 (3H, t, J = 7.2 Hz).

### (Production Example 5)

### (R)-1-{4'-[5-chloro-3-({[1-(thiophen-3-yl)ethoxy]carbonyl}amino)thiophen-2-yl]-[1,1'-biphenyl]-4-yl}cyclopropanecarboxylic acid (Compound No. 1-60)

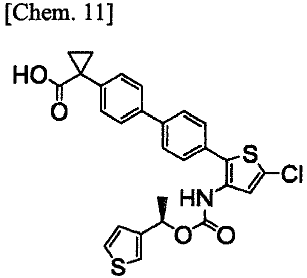

The compound was produced in accordance with the method described in Example 112 of Patent Literature 24 (WO 2014/104372).
Mass spectrum (DUIS⁻, m/z): 522 [M-]⁻.
¹H-NMR spectrum (400 MHz, DMSO-d₆) δ: 12.37 (1H, brs), 9.33 (1H, brs), 7.74-7.68 (2H, m), 7.65-7.60 (2H, m), 7.58-7.50 (3H, m), 7.48-7.37 (3H, m), 7.25-7.07 (2H, m), 5.82 (1H, q, J = 6.4 Hz), 1.56-1.44 (3H, m), 1.48 (2H, dd, J = 6.7, 3.8 Hz), 1.19-1.16 (2H, m).

### (Production Example 6)

### (R)-1-[4'-(5-chloro-3-{[(1-phenylethoxy)carbonyl]amino}thiophen-2-yl)-2'-methoxy-[1 ,1'-biphenyl]-4-yl]cyclopropanecarboxylic acid (Compound No. 1-150)

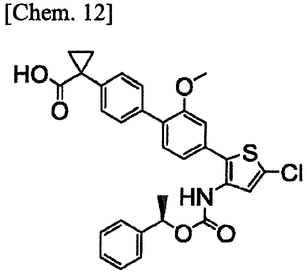

The compound was produced in accordance with the method described in Example 52 of Patent Literature 24 (WO 2014/104372).
Mass spectrum (DUIS⁻, m/z): 546 [M-1]⁻,
¹H-NMR spectrum (400 MHz, DMSO-d₆) δ: 12.34 (1H, brs), 9.40 (1H, brs), 7.45-7.25 (10H, m), 7.21-7.16 (2H, m), 7.09 (1H, dd, J = 7.9, 1.6 Hz), 5.75 (1H, q, J = 6.4 Hz), 3.73 (3H, s), 1.54-1.41 (5H, m), 1.18-1.12 (2H, m).

### (Production Example 7)

### (R)-1-{4'-[5-fluoro-3-({[1-(4-methylthiophen-3-yl)ethoxy]carbonyl}amino)thiophen-2-yl]-[1,1'-biphenyl]-4-yl}cyclopropanecarboxylic acid (Compound No. 1-120)

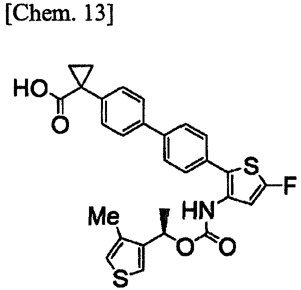

The compound was produced in accordance with the method described in Example 138 of Patent Literature 24 (WO 2014/104372).
Mass spectrum (DUIS⁻, m/z): 520 [M-1]⁻.
¹H-NMR spectrum (400 MHz, DMSO-d₆) δ: 12.38 (1H, brs), 9.33 (1H, brs), 7.73-7.67 (2H, m), 7.65-7.59 (2H, m), 7.57-7.50 (2H, m), 7.49-7.38 (3H, m), 7.19-7.12 (1H, m), 6.83 (1H, brs), 5.74 (1H, q, J = 6.4 Hz), 2.17 (3H, brs), 1.59-1.44 (3H, m), 1.48 (2H, dd, J = 6.7, 3.8 Hz), 1.18 (2H, dd, J = 6.9, 3.9 Hz).

### (Production Example 8)

### (R)-1-{4'-[3-({[1-(2-chlorophenyl)ethoxy]carbonyl}amino)-5-fluorothiophen-2-yl]-2'-methoxy-[1,1'-biphenyl]-4-yl}cyclopropanecarboxylic acid (Compound No. 1-210)

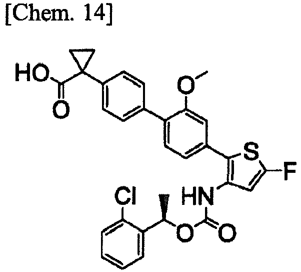

The compound was produced in accordance with the method described in Example 93 of Patent Literature 24 (WO 2014/104372).
Mass spectrum (DUIS⁻, m/z): 564 [M-1]⁻.
¹H-NMR spectrum (400 MHz, DMSO-d₆) δ: 12.35 (1H, brs), 9.55 (1H, brs), 7.60-7.28 (9H, m), 7.18 (1H, d, J = 1.5 Hz), 7.09 (1H, dd, J = 7.8, 1.4 Hz), 6.84 (1H, d, J = 2.5 Hz), 6.00 (1H, q, J = 6.1 Hz), 3.77 (3H, s), 1.55-1.39 (5H, m), 1.21-1.10 (2H, m).

### (Production Example 9)

### (R)-1-{4'-[5-chloro-3-({[1-(2,5-difluorophenyl)ethoxy]carbonyl}amino)thiophen-2-yl]-2'-methoxy-[1,1'-biphenyl]-4-yl}cyclopropanecarboxylic acid (Compound No. 1-170)

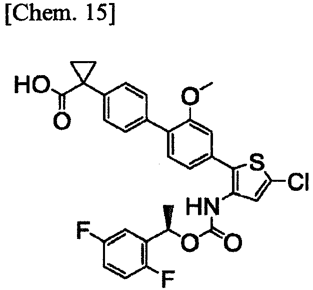

The compound was produced in accordance with the method described in Example 68 of Patent Literature 24 (WO 2014/104372).
Mass spectrum (DUIS⁻, m/z): 582 [M-1]⁻.
¹H-NMR spectrum (400 MHz, DMSO-d₆) δ: 12.35 (1H, brs), 9.54 (1H, brs), 7.44-7.39 (2H, m), 7.39-7.19 (7H, m), 7.18 (1H, d, J = 1.6 Hz), 7.10 (1H, dd, J = 7.9, 1.6 Hz), 5.91 (1H, q, J = 6.5 Hz), 3.76 (3H, s), 1.56-1.43 (3H, m), 1.47 (2H, dd, J = 6.7, 3.7 Hz), 1.18 (2H, dd, J = 6.8, 4.0 Hz).

### [Test Example 1]

### Test of LPA1 antagonism

5 µg of a membrane fraction of RH 7777 cells expressing human LPA1 (A324, ChanTest) was suspended in a reaction buffer (20 mM HEPES, 100 mM NaCl, 10 mM MgCl₂, 10 µM GDP, 5 µg saponin, 0.2% BSA, 0.1 nM [³⁵S]GTPγS (NEG030X, Perkin Elmer), pH 7.4). The test compounds dissolved in DMSO in various concentrations were each added to the suspension. After preincubation at 30°C for 15 minutes, LPA (L7260, Sigma, final concentration 100 nM) was added, and the suspensions were incubated at 30°C for 30 minutes. The membrane fractions were collected on a glass fiber filter (GF/B, Whatman) by using a cell harvester (M30, Brandel), and were washed with a 10 mM phosphate buffer (pH 7.4). The radioactivity of the membrane fractions was measured with a liquid scintillation analyzer (2900TR, Packard). The concentration (IC₅₀) of the test compound required for 50% inhibition of the binding of LPA1 and [³⁵S]GTPγS was determined by non-linear regression analysis using EXSAS (Arm Systex).

In this test, the compounds of the present invention exhibited superior activity, and the IC₅₀ values of the compounds of Production Examples 6, 7 and 8 were not more than 100 nM.

### [Test Example 2]

### Cell migration test

The cell migration test was carried out using Chemo-Tx (registered trademark) (116-8, Neuro Probe). A2058 human melanoma cells (obtained from European Collection of Cell Culture) were cultured in a serum-free EMEM medium for approximately 24 hours, and were re-suspended in a 0.1% BSA-containing DMEM medium to give a cell suspension. The test compounds dissolved in DMSO in various concentrations were each added to the cell suspension, and the suspensions were cultured at 37°C for 15 minutes (final DMSO concentration 0.5%). LPA dissolved in a DMEM medium containing 0.1% BSA and 0.5% DMSO (final LPA concentration 100 nM) was added to a Chemo-Tx 96 well plate, and a Chemo-Tx filter coated with 0.001% Fibronectin on both sides was placed onto the plate. The cultured cell suspensions (50,000 cells) were added onto the upper surface of the filter and were further cultured at 37°C for 3 hours. Thereafter, the cells on the upper surface of the filter were removed. After the filter was removed and was dried, the cells which had migrated to the lower surface of the filter were stained with Diff-Quik stain (16920, Sysmex). The absorbance of the filter (570 nm) was measured. The concentration (IC₅₀) of the test compound required for 50% inhibition of the cell migration activity of LPA was determined by non-linear regression analysis using EXSAS (Arm Systex).

In this test, the compounds of the present invention exhibited superior activity, and the IC₅₀ values of the compounds of Production Examples 5 to 9 were not more than 200 nM.

### [Test Example 3]

### Human hepatic stellate cell α-SMA mRNA expression test

Human primary hepatic stellate cells (obtained from ScienCell Research Laboratories) were suspended in a stellate cell medium containing 2% FBS and 1% stellate cell growth supplement (ScienCell Research Laboratories), and the suspension was seeded on a 24-well plate. The suspension was cultured for 14 hours. Thereafter, the medium was removed, and the stellate cell medium was added and the suspension was further cultured for 3 hours. The medium was removed, and a stellate cell medium containing 0.1% BSA was added. The test compound dissolved in a 0.1% BSA-containing stellate cell medium in various concentrations, and LPA dissolved in a 0.1% BSA-containing PBS (final concentration 10 µM) were added to the suspensions. The suspensions were cultured for 24 hours. Thereafter, the medium was removed, and the stellate cells were lysed by the addition of a lysis buffer (MACHEREY-NAGEL) containing 1 M dithiothreitol.

RNA was extracted from the stellate cell lysates and purified with NucleoSpin (registered trademark) RNA (MACHEREY-NAGEL). cDNA was synthesized using Prime Script (registered trademark) RT reagent Kit with gDNA Eraser (Takara Bio Inc.) and TaKaRa PCR Thermal Cycler Dice (registered trademark) Gradient (Takara Bio Inc.). Quantitative PCR was performed using SYBR (registered trademark) Premix Ex Taq II (Takara Bio Inc.) and Thermal Cycler Dice (registered trademark) Real Time System II (Takara Bio Inc.) to determine the expression levels of α-SMA mRNA and Ribosomal protein, large, P0 mRNA in the stellate cells.

The expression level of α-SMA was corrected with the expression level of ribosomal protein, large, P0, and the inhibition rate (%) of the test compound on the enhanced expression of α-SMA mRNA induced by LPA was determined.

In this test, the compounds of the present invention exhibited superior activity, and the compounds of Production Examples 5 to 9, at a concentration of 100 nM, attained 50% or higher inhibition rate.

### [Test Example 4]

### LPA-induced histamine release test in mice

The LPA-induced histamine release test in mice was carried out in accordance with the method by Swaney et al. (The Journal of Pharmacology and Experimental Therapeutics, 336 (2011), pp. 693-700). The test compound was suspended in a 0.5% methylcellulose solution (133-14255, Wako Pure Chemical Industries, Ltd.), and orally administered to male CD1 mice (body weight 30 to 40 g, supplied by Charles River Laboratories Japan) at a dose of 10 mL/kg. 4 Hours after the administration, LPA (857130P, Avanti) dissolved in 0.1% BSA-containing PBS was administered via the tail vein (300 µg/mouse). Immediately thereafter, each of the mice was anesthetized with isoflurane, and blood was collected from a vein 2 minutes after the administration of LPA. The blood was placed into a test tube containing EDTA, and was centrifuged at 4°C, 2,000 x g for 10 minutes to give plasma.

The histamine concentration in the plasma was measured with an EIA kit (62HTMPEB, Cisbio Bioassays).

The inhibition rate (%) to 0.5% methylcellulose solution administered group was calculated in each individual based on the plasma histamine concentration in the mouse to which the test compound had been administered, and the rate of individuals which showed an inhibition rate of 80% or more was expressed as the efficacy rate (%).

In this test, the compounds of the present invention exhibited superior activity, and the compounds of Production Examples 5 to 9 attained 50% or more efficacy rate at a dose of 10 mg/kg.

### [Test Example 5]

### Non-alcoholic steatohepatitis (NASH) mouse models

STAM (registered trademark) mice (Stelic Institute & Co., Inc.) were used as NASH models. The STAM (registered trademark) mice were prepared by subcutaneously administering 200 µg of streptozotocin (Sigma Aldrich) one time to the back of 2-day old male mice and feeding the mice with a high fat diet (High Fat Diet 32, CLEA Japan, Inc.) from the age of 4 weeks (Medical Molecular Morphology, 46 (2013) pp. 141-152).

The test compound was orally administered every day from the age of 5 or 6 weeks. At the age of 9 or 10 weeks, bloods and livers were collected under anesthesia. The bloods were subjected to biochemical tests. After the wet weights of the livers were measured, RNA was extracted from portions of the livers, and the expression levels of the inflammation and fibrosis marker genes were measured by the quantitative PCR method. Further, the amounts of hydroxyproline or collagen in the livers were measured. Paraffin sections or frozen sections were prepared from portions of the livers and were subjected to histopathological tests to determine the NAFLD activity scores, the fibrosis areas or the inflammation areas. The results were statistically analyzed using EXSUS (CAC EXICARE CORPORATION) or Prism 4 (GraphPad Software, Inc.).

Table 2 shows the results of the above test after 3 weeks of administration of the test compound at 30 mg/kg twice daily from the age of six weeks to the age of nine weeks.

**[Table 2]**

| Production Example (Compound No.) | NAFLD Activity score | |
|---|---|---|
| | Compound administered group | Vehicle group |
| Production Example 6 (I-150) | 2.8±1.0 | 5.1±0.6 |
| Production Example 9 (I-170) | 3.1±1.4 | 4.9±0.6 |

In this test, for example, the compounds of Production Example 6 and Production Example 9 of the present invention exhibited superior activity and attained a significant (p < 0.05) reduction in NAFLD activity score.

From the results of Test Examples 1 to 5, the α-halogenated thiophene compounds of the present invention have LPA1 antagonism and are useful as drugs for the treatment and/or prevention (preferably, for the treatment) of NASH.

Based on the fact that the compounds having LPA1 antagonism have been experimentally shown to be useful for the treatment and/or prevention of NASH, it is assumed that LPA1 antagonists are similarly useful for the treatment and/or prevention of NASH.

### [Preparation Example 1] Hard capsules

Standard two-piece hard gelatin capsules are loaded with a powder (100 mg) of a salt of the compound of Production Example, lactose (150 mg), cellulose (50 mg) and magnesium stearate (6 mg) to give hard capsules, which are washed and then dried.

### [Preparation Example 2] Soft capsules

A mixture of a digestible oil such as soybean oil or olive oil and a salt of the compound of Production Example is injected into gelatin to give soft capsules containing 100 mg of the active ingredient, and the soft capsules are washed and then dried.

### [Preparation Example 3] Tablets

In accordance with a method known in the pharmaceutical field, tablets are produced using a salt of the compound of Production Example (100 mg), colloidal silicon dioxide (0.2 mg), magnesium stearate (0.2 mg), microcrystalline cellulose (0.2 mg), starch (0.2 mg) and lactose (98.8 mg). The tablets may be coated as required.

### INDUSTRIAL APPLICABILITY

The LPA1 antagonists of the present invention, for example, α-halogenated thiophene compounds represented by the general formula (I) or pharmacologically acceptable salts thereof have superior properties such as potent LPA1 antagonism and duration of efficacy, and are useful as drugs for the treatment and/or prevention of NASH.

## Claims

1. A pharmaceutical composition for the treatment and/or prevention of NASH, comprising an LPA1 antagonist as an active ingredient.

2. The pharmaceutical composition for the treatment and/or prevention of NASH according to Claim 1, wherein the LPA1 antagonist is an α-halogenated thiophene compound represented by the general formula (I): 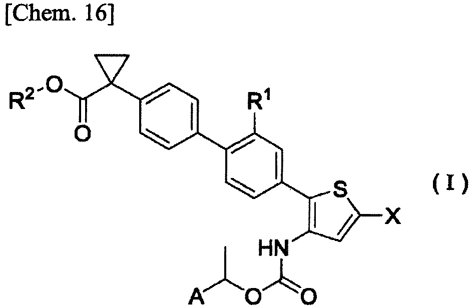 wherein
R¹ is a hydrogen atom or a methoxy group,
R² is a hydrogen atom or a C₁-C₆ alkyl group,
X is a halogen atom, and
A is selected from the group consisting of: 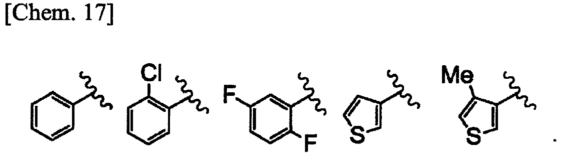
or a pharmacologically acceptable salt thereof.

3. The pharmaceutical composition for the treatment and/or prevention of NASH, comprising an α-halogenated thiophene compound described in Claim 2 wherein X in the general formula (I) is a fluorine atom or a chlorine atom, or a pharmacologically acceptable salt thereof as an active ingredient.

4. The pharmaceutical composition for the treatment and/or prevention of NASH, comprising an α-halogenated thiophene compound described in Claim 3 wherein R¹ in the general formula (I) is a hydrogen atom, or a pharmacologically acceptable salt thereof as an active ingredient.

5. The pharmaceutical composition for the treatment and/or prevention of NASH, comprising an α-halogenated thiophene compound described in Claim 3 wherein R¹ in the general formula (I) is a methoxy group, or a pharmacologically acceptable salt thereof as an active ingredient.

6. A pharmaceutical composition for the treatment and/or prevention of NASH, comprising
(R)-1-[4'-(5-chloro-3-{[(1-phenylethoxy)carbonyl]amino}thiophen-2-yl)-2'-methoxy-[1 ,1'-biphenyl]-4-yl]cyclopropanecarboxylic acid, or a pharmacologically acceptable salt thereof as an active ingredient.

7. A pharmaceutical composition for the treatment and/or prevention of NASH, comprising
(R)-1-{4'-[5-chloro-3-({[1-(2,5-difluorophenyl)ethoxy]carbonyl}amino)thiophen-2-yl]-2'-methoxy-[1,1'-biphenyl]-4-yl}cyclopropanecarboxylic acid, or a pharmacologically acceptable salt thereof as an active ingredient.

8. A pharmaceutical composition for the treatment and/or prevention of NASH, comprising
(R)-1-{4'-[3-({[1-(2-chlorophenyl)ethoxy]carbonyl)amino)-5-fluorothiophen-2-yl]-2'-methoxy-[1,1'-biphenyl]-4-yl}cyclopropanecarboxylic acid, or a pharmacologically acceptable salt thereof as an active ingredient.

9. A pharmaceutical composition for the treatment and/or prevention of NASH, comprising
(R)-1-{4'-[5-chloro-3-({[1-(thiophen-3-yl)ethoxy]carbonyl}amino1,1'-biphenyl]-4-yl}cyclopropanecarboxylic acid, or a pharmacologically acceptable salt thereof as an active ingredient.

10. A pharmaceutical composition for the treatment and/or prevention of NASH, comprising
(R)-1-{4'-[5-fluoro-3-({[1-(4-methylthiophen-3-yl)ethoxy]carbonyl}amino)thiophen-2-yl]-[1,1'-biphenyl]-4-yl}cyclopropanecarboxylic acid, or a pharmacologically acceptable salt thereof as an active ingredient.
